# DEMANDE DE BREVET EUROPEEN

(11) **EP 3 020 450 A1**
(43) Date de publication de la demande: **18.05.2016**
(21) Numéro de dépôt: 15194314.9
(22) Date de dépôt: 12.11.2015
(51) Int. Cl.: A61N 5/06, A61B 5/00, A61B 5/04, A61N 1/36

(54) **DISPOSITIF IMPLANTABLE POUR LA STIMULATION OPTIQUE DU CERVEAU HUMAIN OU ANIMAL**

(30) Priorité: 14.11.2014 FR 1460986
(71) Demandeur: Commissariat à l'Énergie Atomique et aux Énergies Alternatives, 75015 Paris (FR)
(72) Inventeur: CHARVET, Guillaume, 38360 SASSENAGE (FR); BENABID, Alim-Louis, 38240 MEYLAN (FR); CHABROL, Claude, 38320 POISAT (FR); MESTAIS, Corinne, 38660 LA TERRASSE (FR)
(74) Mandataire: Brevalex

(57) **Abrégé**

L'objet principal de l'invention est un dispositif implantable (1) pour la stimulation optique du cerveau humain ou animal, caractérisé en ce qu'il comporte un boîtier (B) destiné à être logé au moins partiellement dans une cavité formée sur le crâne d'un être humain ou animal, un système d'illumination (14) pour la stimulation optique du cerveau, situé dans le boîtier (B), et des moyens électroniques et optiques situés dans le boîtier (B) pour au moins le contrôle de la stimulation optique du cerveau, le boîtier (B) comportant une face inférieure (3b), destinée à être positionnée en vis-à-vis du cerveau, comprenant une zone transparente (Z) pour permettre l'illumination du cerveau.

## Description

### DOMAINE TECHNIQUE

La présente invention se rapporte au domaine des dispositifs implantables au contact du cerveau humain ou animal pour la stimulation (électrique, optique) du cerveau et/ou l'enregistrement de signaux neuronaux. Elle se rapporte tout particulièrement au domaine de la stimulation du cerveau par irradiation optique.

L'invention peut concerner les dispositifs implantables d'illumination du cortex cérébral et les implants cérébraux connus sous l'appellation d' « interface neuronale » permettant une communication entre le cerveau et un dispositif électronique, notamment la mesure de signaux électriques neuronaux enregistrés par des électrodes placées au niveau du cerveau.

L'invention propose ainsi un dispositif implantable pour la stimulation optique du cerveau d'un être humain ou animal.

### ÉTAT DE LA TECHNIQUE ANTÉRIEURE

De nombreux systèmes implantables dans le cerveau humain ou animal pour l'illumination du cerveau et le traitement de signaux neuronaux, notamment la lecture, l'enregistrement et/ou le contrôle de signaux neuronaux, ont été conçus depuis ces dernières années afin notamment de diagnostiquer, de prévenir, d'assister, d'améliorer ou de réparer des fonctions humaines ou animales de cognition ou des actions défaillantes.

De tels systèmes ont par exemple trouvé des applications dans le traitement de crises d'épilepsie, dans l'atténuation des symptômes de la maladie de Parkinson, dans la lutte contre la dépression et dans le traitement d'autres problèmes médicaux.

Parmi ces systèmes implantables, on distingue les systèmes implantables d'illumination du cortex cérébral. De tels systèmes visent à stimuler optiquement le cerveau humain ou animal afin d'en obtenir un effet bénéfique notamment sur le traitement de maladies.

On peut ainsi citer par exemple les dispositifs implantables de stimulation optique divulgués dans le document intitulé « From Optogenetic Technologies to Neuromodulation Therapies », Justin C. Williams et al, 20 mars 2013, Science translational medicine, Volume 5 (177), p. 177 ps6, et dans la demande de brevet américain US 2011/0125077 A1.

En outre, il existe également les systèmes implantables sous la forme d'implants cérébraux connus sous l'appellation d' « interface neuronale ». Ces systèmes peuvent notamment permettre le pilotage d'effecteurs pour des applications dites de type « Interface Neuronale Directe » (IND) ou « Brain-Computer Interface » (BCI) en anglais (interface cerveau-machine). De façon générale, les applications de type IND ou BCI peuvent permettre le pilotage d'actionneurs au moyen des signaux neuronaux collectés par des électrodes implantées dans ou sur le cerveau.

Les divers implants cérébraux connus de l'art antérieur pour le traitement de signaux neuronaux peuvent être classifiés en fonction de la zone d'implantation.

La technologie de l'électrocorticographie (EcoG) caractérise les dispositifs permettant l'enregistrement graphique de l'activité cérébrale grâce à des électrodes, réparties selon une matrice, placées au contact direct du cortex sous ou sur la dure-mère. Les signaux corticaux mesurés sont traités par des moyens électroniques. Cette technique est invasive car elle requiert d'effectuer une craniotomie sur l'individu à examiner, c'est-à-dire une incision chirurgicale au niveau du crâne.

Enfin, on connaît également deux autres technologies de mesure des signaux neuronaux, et en particulier du potentiel neuronal, connues sous les appellations de « Single Unit Action Potential » (SUAP) et « Local Field Potential » (LFP) en anglais. Elles reposent sur le principe de pénétration d'électrodes en pointes dans le cerveau, en particulier dans le cortex pour y effectuer la mesure.

Les moyens électroniques permettant de recevoir les signaux neuronaux captés par les électrodes peuvent être situés à l'extérieur du cerveau et reliés aux électrodes par des liaisons filaires. Toutefois, pour optimiser le rapport signal sur bruit, il s'est avéré intéressant de vouloir les placer au plus près de la zone d'enregistrement des signaux et ainsi de les implanter également au niveau du cerveau.

L'article intitulé « BioBolt : A Minimally-Invasive Neural Interface for Wireless Epidural Recording by Intra-Skin Communication », Sun-II Chang et al, 2011 Symposium on VLSI Circuits Digest of Technical Papers, pages 146-147, et la demande internationale WO 2011/123150 A1 décrivent une interface neuronale qui comporte une tête élargie placée au sommet d'un corps fileté. La tête est destinée à venir s'appliquer contre le crâne alors que le corps fileté est prévu pour être inséré dans un alésage de même diamètre formé dans l'os du crâne. Une électrode est disposée à la base du corps fileté, et couplée électriquement à un circuit électrique situé dans la tête élargie. Le circuit électrique peut permettre une éventuelle pré-amplification et/ou une numérisation des signaux électriques collectés par l'électrode, et leur transmission vers des moyens électroniques ou informatiques distants, par une liaison filaire ou hertzienne, pour leur traitement.

Par ailleurs, on connait également des implants cérébraux munis d'une fenêtre en saphir, utilisée pour la communication de données par radiofréquence ou par infrarouge, tels que décrits dans le document intitulé « An implantable wireless neural interface for recording cortical circuit dynamics in moving primates », David A. Borton et al, 2013, Journal of neural engineering, Volume 10 (2), 026010.

Les systèmes implantables d'illumination du cortex cérébral et les implants cérébraux décrits ci-dessus, qui relèvent de techniques d'implantation invasives, sont des dispositifs placés au contact direct de la peau, celle-ci recouvrant le plus souvent la partie supérieure des dispositifs. Ce contact direct avec la peau peut engendrer des risques de lésions et/ou d'infection de la peau non négligeables, et ce plus particulièrement au niveau de la jonction entre le crâne et la partie supérieure du dispositif lorsque ce dernier est logé dans une cavité du crâne formée par craniotomie. De plus, l'intégration de ces systèmes et implants sur le cerveau d'un individu peut entraîner une gêne importante pour l'individu au niveau de la zone d'implantation liée à son confort d'utilisation. Elle peut également s'avérer disgracieuse et révéler un aspect extérieur du crâne non homogène. En outre, les systèmes implantables connus d'illumination ne permettent généralement pas de réaliser une illumination du cortex sur une grande surface.

La demande de brevet EP 2 623 025 A1 de la Demanderesse propose déjà un dispositif implantable du type interface neuronale permettant une implantation facilitée sur un individu tout en minimisant les risques médicaux. Toutefois, ce dispositif n'est pas prévu pour permettre la stimulation optique du cortex cérébral de l'individu.

### EXPOSÉ DE L'INVENTION

Il existe par conséquent un besoin pour prévoir un dispositif implantable qui permette de réaliser une stimulation optique du cerveau sur une grande surface, notamment une large zone du cortex cérébral. Il existe également un besoin pour concevoir un tel dispositif implantable permettant de minimiser les risques médicaux liés à son utilisation, notamment les risques de lésions et/ou d'infection du corps d'un individu, et notamment de la peau recouvrant le crâne de l'individu.

Il existe en outre un besoin pour permettre une intégration homogène d'un tel dispositif au niveau du cerveau afin d'obtenir un confort esthétique et physique pour l'individu.

L'invention a pour but de remédier à tout ou partie des besoins mentionnés ci-dessus et aux inconvénients relatifs aux réalisations de l'art antérieur.

L'invention a ainsi pour objet, selon l'un de ses aspects, un dispositif implantable pour la stimulation optique du cerveau humain ou animal, caractérisé en ce qu'il comporte :
- un boîtier destiné à être logé au moins partiellement dans une cavité formée sur le crâne d'un être humain ou animal,
- un système d'illumination pour la stimulation optique du cerveau de l'être humain ou animal, situé dans le boîtier, et
- des moyens électroniques et optiques situés dans le boîtier pour au moins le contrôle de la stimulation optique du cerveau,
le boîtier comportant une face inférieure, destinée à être positionnée en vis-à-vis du cerveau de l'être humain ou animal, comprenant une zone transparente pour permettre l'illumination du cerveau.

Grâce à l'invention, le dispositif implantable peut permettre de générer une stimulation optique sur une surface importante du cerveau, notamment sur une zone de plus grande dimension caractéristique, par exemple le diamètre, supérieure ou égale à 1 cm, de préférence supérieure ou égale à 3 cm. De plus, le dispositif peut être intégré de façon homogène au niveau du crâne de l'individu et présenter un nombre nul ou sensiblement nul d'aspérités sur sa face supérieure au contact de la peau de sorte à ne pas entraîner de risques de lésions et/ou d'infection de la peau. Le dispositif peut également permettre une incorporation dans la continuité du crâne, en réduisant fortement la cassure habituelle qui existe à la jonction entre le crâne et la face supérieure du dispositif, de sorte que le dispositif soit le plus discret possible et tende à être visuellement indétectable. Autrement dit, le dispositif implantable selon l'invention peut permettre de minimiser les risques médicaux lors de son implantation tout en assurant le confort esthétique et physique de l'individu.

Par ailleurs, l'illumination du cerveau par le dispositif implantable selon l'invention, en particulier dans l'infrarouge ou le proche infrarouge, peut permettre le traitement de maladies neurodégénératives, telles que la maladie d'Alzheimer. Des expérimentations ont en effet été menées sur des animaux et ont démontré qu'une irradiation de certaines parties du cerveau animal par la lumière infrarouge ou proche infrarouge peut avoir un effet bénéfique sur le développement de maladies, telles que la maladie de Parkinson, si bien qu'une telle irradiation peut avoir un effet bénéfique neuroprotecteur sur l'évolution des maladies neurodégénératives.

Le dispositif implantable selon l'invention peut en outre comporter l'une ou plusieurs des caractéristiques suivantes prises isolément ou suivant toutes combinaisons techniques possibles.

La zone transparente de la face inférieure du boîtier peut s'étendre sur toute la surface de la face inférieure.

La zone transparente de la face inférieure peut préférentiellement être réalisée en saphir. De façon avantageuse, le saphir est un matériau présentant des propriétés de transparence, de solidité et de biocompatibilité tout à fait satisfaisantes pour les besoins de l'invention.

Le système d'illumination peut comporter une ou plusieurs sources lumineuses destinées à illuminer le cortex cérébral de l'être humain ou animal.

La ou les sources lumineuses sont préférentiellement de faible consommation électrique.

La ou les sources lumineuses peuvent être choisies parmi des diodes électroluminescentes (ou encore LED pour « Light-Emitting Diode » en anglais), des diodes électroluminescentes organiques (ou encore OLED pour « Organic Light-Emitting Diode » en anglais), des diodes laser, des diodes laser à cavité verticale émettant par la surface (ou encore VCSEL pour « Vertical-Cavity Surface Emitting Laser » en anglais) et/ou une matrice de telles diodes.

La ou les sources lumineuses peuvent être conçues pour permettre différents modes d'utilisation, et notamment pour permettre une illumination continue sur toute la surface couverte par la ou les sources lumineuses ou sur une partie locale, une illumination non continue à une fréquence prédéterminée sur toute la surface couverte par la ou les sources lumineuses ou sur une partie locale, ou encore une illumination contrôlée selon des paramètres donnés, tels que l'enregistrement de l'activité neuronale par exemple.

La ou les sources lumineuses peuvent s'étendre sur toute la surface de la zone transparente de la face inférieure du boîtier.

La ou les sources lumineuses peuvent de manière préférée émettre dans l'infrarouge et/ou le proche infrarouge, la plupart des tissus biologiques présentant un coefficient de transmission élevé dans cette plage de longueurs d'ondes.

La zone transparente de la face inférieure du boîtier peut comporter un diffuseur, notamment un diffuseur intermédiaire ou structuré, pour améliorer l'uniformité d'illumination du cerveau, ou un dispositif optique permettant de focaliser la lumière sur des zones précises.

La zone transparente de la face inférieure peut être équipée de lentilles ou d'une matrice de microlentilles pour focaliser le faisceau lumineux dans l'intention de stimuler localement et préférentiellement certaines zones du cortex.

Tout ou partie des sources lumineuses peuvent être optiquement couplées à une optique de focalisation. Des moyens peuvent permettre le déplacement relatif d'une ou de chaque source lumineuse par rapport à l'optique de focalisation à laquelle elle est associée.

Chaque source lumineuse est apte à produire un rayonnement continu, ou impulsionnel, la fréquence étant alors comprise entre quelques hertz (Hz) et quelques centaines de kilohertz (kHz). Chaque source lumineuse peut être modulée en amplitude selon une fréquence prédéterminée.

De plus, la zone transparente de la face inférieure du boîtier peut présenter une plus grande dimension caractéristique, notamment un diamètre, supérieure ou égale à 1 cm, de préférence supérieure ou égale à 3cm pour permettre une illumination surfacique du cerveau.

La ou les sources lumineuses du système d'illumination peuvent être solidaires d'un moyen de déplacement, en particulier selon un plan parallèle ou sensiblement parallèle à la face inférieure du boîtier, notamment un moyen de translation, et en particulier un actuateur piézo-électrique, permettant le commandement du déplacement d'une source ou de plusieurs sources lumineuses. Un tel moyen de déplacement peut par exemple permettre un débattement d'une centaine à quelques centaines de micromètres selon une direction donnée, en particulier parallèlement ou sensiblement parallèlement à la face inférieure du boîtier.

Par ailleurs, le dispositif selon l'invention peut être du type interface neuronale pour le traitement de signaux, notamment des signaux neuronaux, les moyens électroniques et optiques situés dans le boîtier étant configurés en outre pour le traitement de signaux, notamment de signaux neuronaux, et en particulier pour la stimulation optique et/ou électrique et l'enregistrement de signaux électriques. Le dispositif peut de plus comporter un système d'électrodes couplé électriquement aux moyens électroniques.

Autrement dit encore, les moyens électroniques et optiques peuvent comporter un module de traitement de signaux, notamment de signaux neuronaux, et notamment un module de stimulation électrique et d'enregistrement de signaux électriques.

Par ailleurs, les moyens électroniques et optiques peuvent avantageusement comporter un module de communication avec un dispositif externe, notamment un module de communication sans fil avec un terminal distant pour le contrôle du fonctionnement du dispositif et/ou la transmission de données.

De plus, les moyens électroniques peuvent également comporter un module d'alimentation, notamment du système d'illumination.

Le système d'électrodes peut préférentiellement comporter une matrice d'électrodes transparentes, permettant la mesure de signaux corticaux, tout en permettant l'illumination du cerveau par le système d'illumination.

Le système d'électrodes peut être fixé sous le boîtier, au niveau de la face inférieure de la partie inférieure du boîtier.

Le système d'électrodes peut être inscrit dans le périmètre externe du boîtier, notamment dans le périmètre externe de la partie inférieure du boîtier.

Le système d'électrodes peut comporter des électrodes agencées selon une matrice prédéterminée, notamment en un réseau régulier ou non.

En outre, le système d'électrodes peut comporter des électrodes d'enregistrement de signaux neuronaux et/ou des électrodes de stimulation. Les électrodes de stimulation peuvent par exemple permettre l'émission de signaux électriques dans le cerveau, par exemple pour traiter différents maux tels que la maladie de Parkinson, la dépression, la migraine, l'obésité et/ou la douleur, entre autres.

Les moyens électroniques et optiques peuvent comporter des moyens de traitement de signaux neuronaux mesurés par les électrodes d'enregistrement et/ou des moyens de production de signaux d'électrostimulation par les électrodes de stimulation.

Par ailleurs, le dispositif selon l'invention peut comporter au moins une antenne de transmission (ou encore de communication), pour l'échange d'informations en émission et/ou réception, et/ou une antenne de téléalimentation.

Le boîtier peut comporter un moyen d'alimentation tel qu'une batterie rechargeable ou une pile, pouvant le cas échéant être alimentée par ladite au moins une antenne de téléalimentation.

Ladite au moins une antenne de transmission et/ou ladite au moins une antenne de téléalimentation peuvent être situées dans le boîtier.

La disposition à l'intérieur du boîtier de l'antenne de transmission et/ou de l'antenne de téléalimentation peut notamment être choisie lorsque le boîtier est réalisé en céramique.

En variante, ladite au moins une antenne de transmission et/ou ladite au moins une antenne de téléalimentation peuvent être situées à l'extérieur du boîtier.

La disposition à l'extérieur du boîtier de l'antenne de transmission et/ou de l'antenne de téléalimentation peut notamment être choisie lorsque le boîtier est réalisé en titane.

Le dispositif selon l'invention peut par ailleurs comporter une enveloppe de transmission de signaux, s'étendant depuis la périphérie du boîtier, notamment depuis la périphérie de la partie inférieure du boîtier.

Par « transmission de signaux », on entend la transmission de signaux mesurés (neuronaux, optiques) et/ou de signaux stimulation (électrique, optique), et/ou la transmission de signaux à destination des moyens électroniques et optiques, par exemple des signaux de téléalimentation et/ou des signaux de commande pilotant ces moyens.

L'enveloppe peut comporter ladite au moins une antenne de transmission et/ou ladite au moins une antenne de téléalimentation.

En particulier, l'enveloppe peut comporter une antenne de transmission et une antenne de téléalimentation, les deux antennes étant coplanaires et disposées de sorte que l'une est inscrite dans l'autre.

L'enveloppe peut être réalisée dans un matériau souple et biocompatible, notamment à partir d'un élastomère de type silicone.

L'enveloppe peut présenter une épaisseur suffisamment mince pour pouvoir être glissée entre le crâne et la peau du cuir chevelu.

L'épaisseur de l'enveloppe peut notamment diminuer en éloignement du boîtier. Autrement dit, l'épaisseur de l'enveloppe peut diminuer avec la distance par rapport à la périphérie du boîtier. L'épaisseur de l'enveloppe peut par exemple être comprise entre 1 et 3 mm, étant par exemple inférieure à 1,5 mm, mieux 1 mm, mieux encore 0,7 mm. L'épaisseur de l'enveloppe peut être constante sur une distance prédéterminée à partir du boîtier, puis diminuer progressivement jusqu'à l'extrémité de l'enveloppe.

L'enveloppe peut présenter une dureté Shore A inférieure ou égale à 50.

L'enveloppe peut présenter une surface supérieure sensiblement convexe (ou courbe) de façon à reproduire au moins partiellement la courbure du crâne et/ou celle du cuir chevelu à l'endroit de son implantation, et à assurer une sensible continuité de surface entre la face supérieure du boîtier et la surface supérieure de l'enveloppe.

En particulier, la surface supérieure de l'enveloppe peut être reliée à la face supérieure du boîtier de façon continue de sorte à présenter un arrondi épousant la forme du cuir chevelu et/ou celle du crâne à l'interface entre le boîtier et l'enveloppe.

La surface supérieure de l'enveloppe et la face supérieure du boîtier peuvent être tangentes l'une par rapport à l'autre au niveau de leur interface.

En outre, le boîtier peut comporter une face supérieure, destinée à venir au contact de la peau de la tête de l'être humain ou animal, dont la surface est sensiblement convexe.

Par « sensiblement convexe », on entend que la surface de la face supérieure est convexe, ou tout au moins apparaît comme convexe à l'oeil nu.

La convexité de la surface de la face supérieure du boîtier peut être déterminée de façon à reproduire au moins partiellement la courbure du crâne au niveau de ladite cavité et à assurer une sensible continuité de surface entre la face supérieure du boîtier et les portions de surface du crâne adjacentes. La courbure de la face supérieure du boîtier peut par exemple être équivalente à la courbure réelle du crâne de l'être humain ou animal, une telle courbure variant en fonction du crâne et de la zone d'implantation. Le rayon de courbure peut ainsi varier entre 50 et 200 mm.

Le boîtier peut être formé par l'assemblage d'une partie supérieure et d'une partie inférieure, notamment par soudage.

La partie supérieure peut comporter un rebord s'étendant depuis la face supérieure du boîtier et définissant la surface latérale de la partie supérieure, le rebord étant destiné à être inséré dans la partie inférieure du boîtier, en prenant appui contre la face latérale de la partie inférieure.

Le rebord peut s'étendre sur tout le pourtour de la partie supérieure.

La partie supérieure du boîtier peut comporter des pattes de fixation, faisant notamment saillies sur le rebord, destinées à prendre appui sur le crâne de l'être humain ou animal.

La partie supérieure peut présenter une forme sensiblement circulaire ou polygonale, notamment une forme carrée.

Des méplats peuvent être formés au niveau d'angles, notamment de chaque angle, de la partie supérieure, les pattes de fixation pouvant notamment faire saillies à partir de ces méplats.

La partie inférieure peut présenter une face inférieure et une face latérale qui définissent entre elles un logement en forme de cuvette pour recevoir les moyens électroniques.

La face latérale de la partie inférieure peut être ajourée, comportant notamment des ouvertures pour permettre le passage de contacts électriques pour coupler électriquement les moyens électroniques et optiques avec le système d'électrodes.

La partie inférieure peut présenter une forme sensiblement circulaire ou polygonale, notamment une forme carrée.

Le boîtier, notamment la partie supérieure et/ou la partie inférieure, peut être surmoulé par un matériau de surmoulage, notamment une résine de silicone biocompatible.

Le boîtier peut présenter une plus grande dimension transversale comprise entre 1 et 5 cm. De plus, l'épaisseur du boîtier peut être comprise entre 0,3 et 1 cm, notamment entre 0,5 et 1 cm.

Le boîtier, notamment la partie supérieure et/ou la partie inférieure, et en particulier la face inférieure du boîtier, peut être réalisé dans un matériau rigide et biocompatible, étant par exemple réalisé en titane, en céramique ou en saphir transparent.

Le boîtier peut être hermétique, afin notamment d'éviter toute intrusion de liquide à l'intérieur du boîtier.

L'invention a encore pour objet, selon un autre de ses aspects, un procédé d'implantation d'un dispositif tel que défini précédemment dans une cavité formée sur le crâne d'un être humain ou animal.

Selon ce procédé d'implantation du dispositif selon l'invention, on peut placer le dispositif dans la cavité de sorte que la face supérieure du boîtier et les portions de surface du crâne adjacentes au boîtier forment sensiblement une continuité de surface.

Par « continuité de surface », on entend que la jonction entre la face supérieure du dispositif, notamment celle du boîtier, et les portions de surface du crâne adjacentes ne présente sensiblement pas de surépaisseur. En particulier, la jonction entre la face supérieure du dispositif et le crâne peut présenter un aspect lisse. Ainsi, d'une façon générale, au niveau de ladite jonction, le plan tangent à la face supérieure du dispositif est parallèle, voire sensiblement confondu, au plan tangent au crâne dans lequel la face supérieure est insérée.

En outre, selon ce procédé d'implantation du dispositif selon l'invention, on peut placer la face inférieure du boîtier face à la dure-mère, celle-ci jouant le rôle de diffuseur de lumière.

L'invention a encore pour objet, selon un autre de ses aspects, un procédé de fabrication d'un dispositif, et notamment d'un boîtier, tel que défini précédemment.

Selon ce procédé de fabrication du dispositif selon l'invention, on peut déterminer la convexité de la face supérieure du boîtier du dispositif à partir de mesures de convexité préalablement effectuées sur le crâne d'un être humain ou animal.

En outre, selon ce procédé de fabrication du dispositif selon l'invention, on peut réaliser la liaison entre la partie transparente, notamment réalisée en saphir, de la face inférieure du boîtier et le boîtier, notamment réalisé en titane, par le biais d'une brasure hermétique, en particulier une brasure hermétique or. Un enrobage réalisé avec un matériau biocompatible, par exemple une silicone biocompatible, permet d'éviter un contact direct entre l'or et le milieu physiologique.

Le dispositif implantable et les procédés d'implantation et de fabrication selon l'invention peuvent comporter l'une quelconque des caractéristiques énoncées dans la description, prises isolément ou selon toutes combinaisons techniquement possibles avec d'autres caractéristiques.

### BRÈVE DESCRIPTION DES DESSINS

L'invention pourra être mieux comprise à la lecture de la description détaillée qui va suivre, d'exemples de mise en oeuvre non limitatifs de celle-ci, ainsi qu'à l'examen des figures, schématiques et partielles, du dessin annexé, sur lequel :
- la figure 1 est une représentation, en perspective et en vue éclatée, d'un exemple de dispositif implantable selon l'invention,
- la figure 2 représente, en perspective, le dispositif de la figure 1 dans une configuration assemblée,
- la figure 3 représente, en perspective, la partie supérieure du boîtier du dispositif des figures 1 et 2,
- la figure 4 est une vue en coupe et en perspective du dispositif des figures 1 et 2,
- la figure 5 représente, en perspective, la partie inférieure du boîtier du dispositif des figures 1 et 2,
- la figure 6 représente, en perspective et en vue du dessous, le dispositif des figures 1 et 2,
- la figure 7 représente, en vue de face, le système d'électrodes du dispositif des figures 1 et 2,
- les figures 8a et 8b illustrent deux étapes de la fabrication de l'enveloppe du dispositif de la figure 1,
- les figures 9 et 10 illustrent, respectivement en vue en perspective et en coupe, l'implantation de deux dispositifs selon l'invention sur le crâne d'un être humain, et
- les figures 11, 12 et 13 sont des représentations, en vue de dessous, de trois variantes de réalisation de dispositifs implantables conformes à l'invention.

Dans l'ensemble de ces figures, des références identiques peuvent désigner des éléments identiques ou analogues.

De plus, les différentes parties représentées sur les figures ne le sont pas nécessairement selon une échelle uniforme, pour rendre les figures plus lisibles.

### EXPOSÉ DÉTAILLÉ DE MODES DE RÉALISATION PARTICULIERS

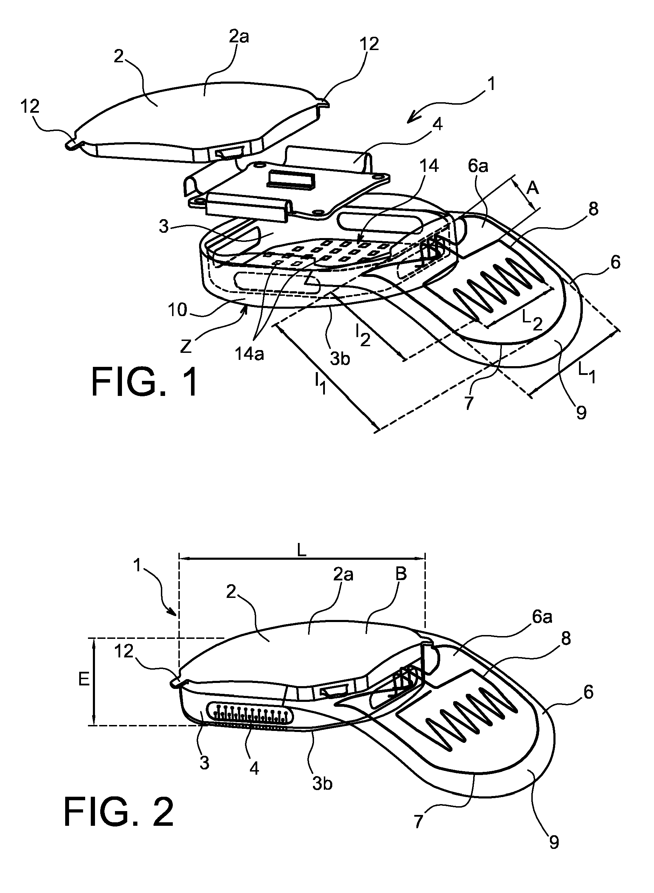

On a représenté sur les figures 1 et 2 un exemple de dispositif implantable 1 conforme à l'invention pour la stimulation optique du cerveau d'un être humain ou animal.

Le dispositif 1 comporte un boîtier B à l'intérieur duquel sont logés un système d'illumination 14 pour la stimulation optique du cerveau de l'être humain ou animal, et des moyens électroniques et optiques 4 pour le contrôle de la stimulation optique du cerveau. Le système d'illumination 14 est connecté à des cartes électroniques, non représentées, permettant son alimentation et sa commande.

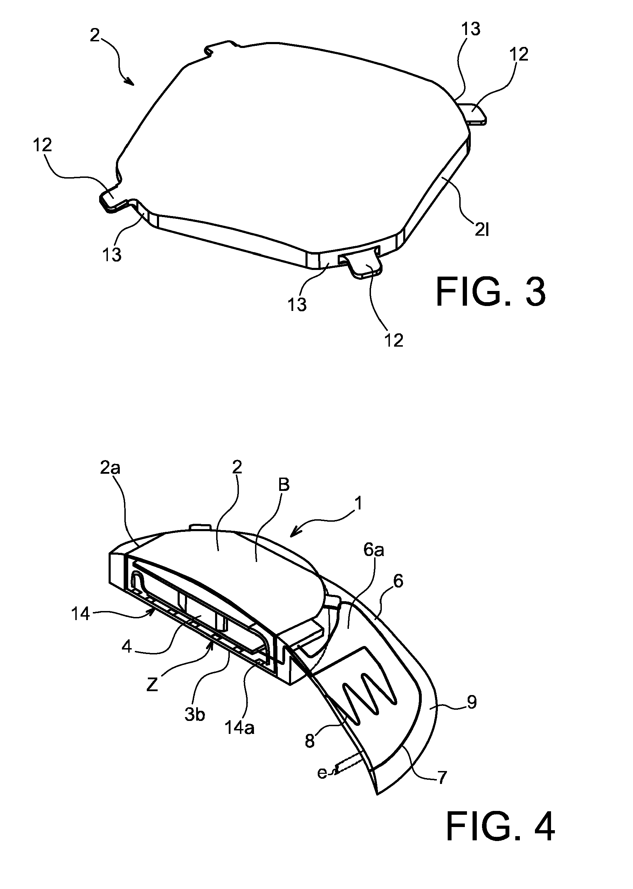

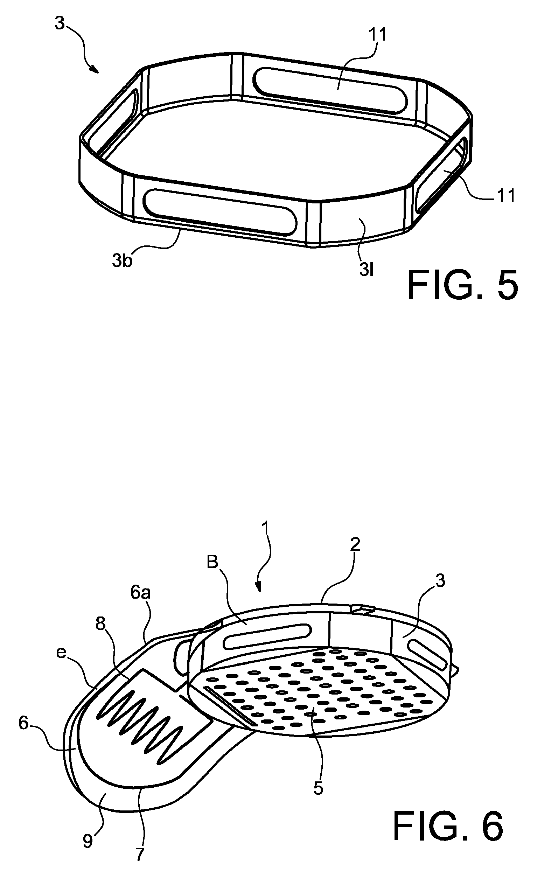

Le boîtier B est formé par l'assemblage d'une partie supérieure 2 et d'une partie inférieure 3, respectivement visibles de manière isolée sur les figures 3 et 5.

Le boîtier B est avantageusement hermétique, afin d'éviter toute intrusion de liquide voire de gaz, et biocompatible. Il est réalisé en outre dans un matériau rigide, et en particulier en céramique ou en titane. De plus, il présente une face inférieure 3b de diamètre égal à environ 5 cm, ce qui permet d'obtenir une illumination de grande surface. La face inférieure 3b est plane, ou présente une légère courbure de façon à épouser la forme du cortex.

Conformément à l'invention, le boîtier B comporte une face inférieure 3b, face inférieure de la partie inférieure 3, destinée à être positionnée en vis-à-vis du cerveau de l'être humain ou animal, qui comprend une zone transparente Z pour permettre l'illumination du cerveau. Cette zone transparente Z est avantageusement réalisée en saphir et s'étend sur toute la surface de la face inférieure 3b.

Le choix du saphir pour cette zone transparente Z permet de lui conférer de très bonnes propriétés de transparence, de solidité et de biocompatibilité.

Cette zone de transparence Z correspond avantageusement à une fenêtre transparente au travers de laquelle l'irradiation optique du système d'illumination 14 peut passer pour atteindre le cortex cérébral de l'être humain ou animal. La reprise de contact entre la fenêtre transparente Z en saphir et le boîtier B en titane ou céramique permet d'assurer l'herméticité du boîtier B.

Par ailleurs, comme on peut le voir sur la figure 1, le système d'illumination 14 comporte une pluralité de sources lumineuses 14a pour illuminer le cortex cérébral de l'être humain ou animal. Ces sources lumineuses 14a peuvent être choisies parmi des diodes LED, OLED, laser, VCSEL ou une matrice de telles diodes.

Ces sources lumineuses 14a sont en superposition de la surface de la face inférieure 3b comprenant en totalité la zone transparente 2.

De façon avantageuse, ces sources lumineuses 14a émettent dans l'infrarouge et/ou le proche infrarouge.

Par ailleurs, bien que non représenté, la zone transparente Z peut comporter un diffuseur, notamment un diffuseur intermédiaire ou structuré, pour améliorer l'uniformité d'illumination du cerveau. La dure-mère peut également permettre le cas échéant d'améliorer la diffusion de la lumière vers le cortex cérébral.

Le système d'illumination 14 peut en particulier jouer deux fonctions en fonction de l'application visée. D'une part, il peut permettre d'obtenir une stimulation optique surfacique avec un « champ large », soit une grande surface d'illumination, du cortex, de plus grande dimension caractéristique supérieure ou égale à 5 cm. D'autre part, il peut permettre d'obtenir une stimulation optique focalisée du type « multipoints » de sorte à stimuler une zone précise du cortex cérébral. Cette stimulation peut se faire au moyen d'une matrice de sources lumineuses 14a déterminée en fonction des besoins.

En outre, le dispositif 1 est également du type interface neuronale. Les moyens électroniques et optiques 4 sont ainsi configurés pour le traitement de signaux neuronaux, et en particulier pour la stimulation et l'enregistrement de signaux neuronaux.

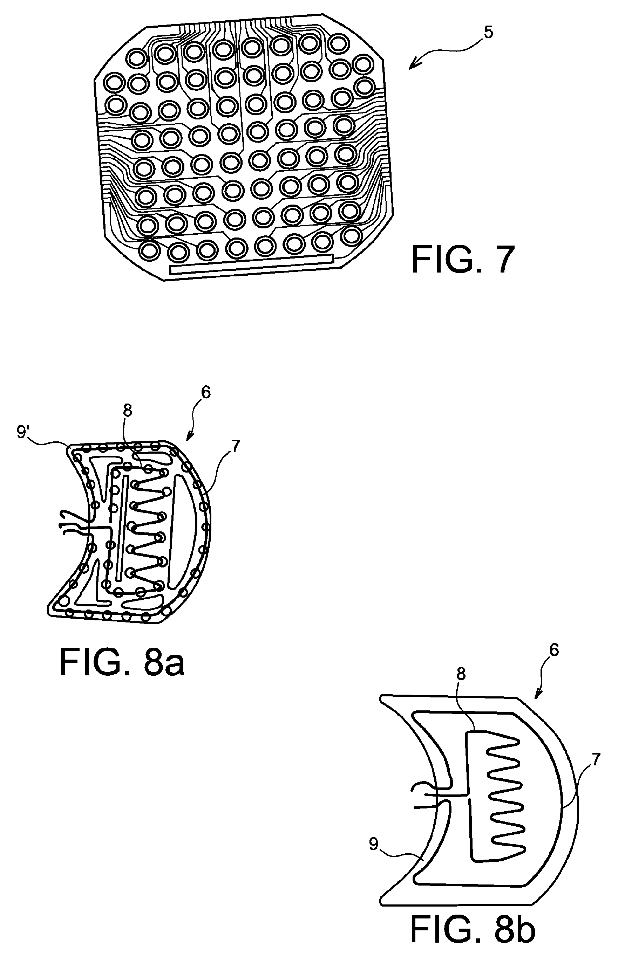

Le dispositif 1 comporte de plus un système d'électrodes 5 couplé électriquement aux moyens électroniques et optiques 4, comme on peut le voir sur la figure 6 et de façon isolée sur la figure 7.

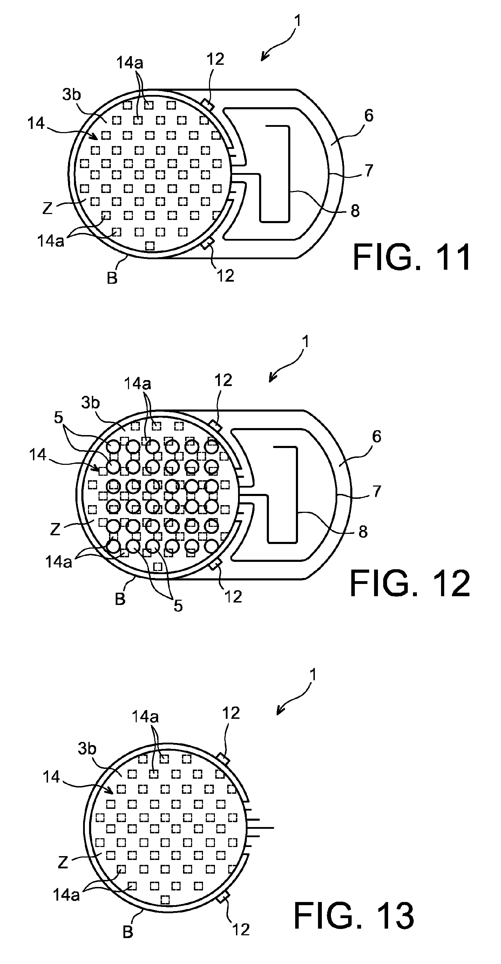

Ce système d'électrodes 5 peut avantageusement comporter un réseau de matrice d'électrodes transparente 5 pour permettre l'illumination du cerveau par le système d'illumination 14. Un tel exemple de réalisation est encore illustré et décrit par la suite en référence à la figure 12.

Par ailleurs, le dispositif 1 comporte encore une enveloppe 6 de transmission, qui sera détaillée ultérieurement.

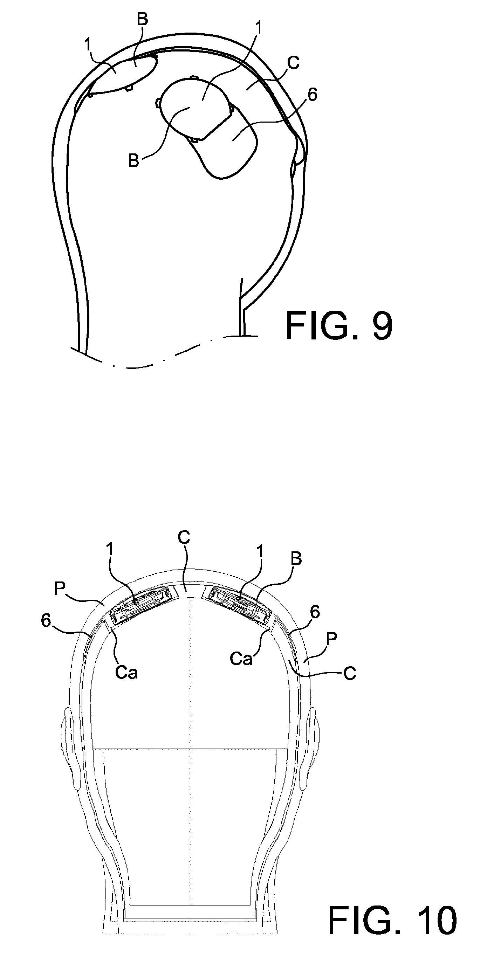

Le dispositif implantable 1, et notamment le boîtier B du dispositif 1, est destiné à être implanté au contact du cerveau d'un individu afin notamment d'enregistrer et de traiter des signaux neuronaux du cerveau. Les figures 9 et 10 illustrent, de façon schématique, l'implantation de deux dispositifs 1 conforme à l'invention respectivement dans deux cavités Ca formées sur le crâne C d'un être humain, par exemple à la suite d'une opération de craniotomie. Le boîtier B d'un dispositif 1 est logé dans une cavité Ca alors que l'enveloppe 6 de ce dispositif 1 est insérée entre le crâne C et la peau P du cuir chevelu.

Le boîtier B comporte par ailleurs une face supérieure 2a, destinée à venir au contact de la peau P, dont la surface est sensiblement convexe. La convexité de la surface de la face supérieure 2a peut permettre de conférer à la partie supérieure 2 du boîtier B destinée à venir au contact de la peau P une forme arrondie de façon à reproduire au moins partiellement, voir totalement, la courbure du crâne C au niveau de la cavité Ca formée dans celui-ci et, par ailleurs, d'assurer une sensible continuité de surface entre la face supérieure 2a du boîtier B et les portions de surface du crâne C adjacentes au dispositif 1. La convexité de la face supérieure 2a peut donc permettre un lissage de l'interface entre le dispositif 1 et les portions de surface du crâne C qui lui sont adjacentes.

Avantageusement, la face supérieure 2a du boîtier B peut permettre d'épouser la forme du crâne C à l'endroit de la cavité Ca formée dans celui-ci de façon à réduire, voire à rendre inexistantes, les aspérités sur la surface de la face supérieure 2a du boîtier B, et en particulier au niveau de la jonction entre la face supérieure 2a du boîtier B et les portions de surface du crâne C adjacentes au dispositif 1. De la sorte, le risque de liaisons et/ou d'infection de la peau, notamment au niveau de cette jonction, est fortement réduit. Le dispositif 1 peut donc être implanté au contact du cerveau pour une longue durée tout en limitant les risques pour l'individu. La convexité de la face supérieure 2a du boîtier B peut en effet permettre au dispositif 1, et notamment à la face supérieure 2a de la partie supérieure 2 du boîtier B, de venir à affleurement de l'os du crâne C situé à la périphérie de la cavité Ca.

La face supérieure 2a du boîtier B peut présenter une surface convexe ou multiconvexe, en particulier biconvexe, par exemple ayant une convexité selon deux directions orthogonales. La convexité de la surface de la face supérieure 2a du boîtier B peut par exemple être déterminée par rapport à la courbure du crâne C, notamment au niveau de la cavité Ca avant sa formation, par exemple par craniotomie. En particulier, la face supérieure 2a du boîtier B peut présenter sensiblement la même courbure que celle du crâne C dans la région où la cavité Ca est formée.

La partie supérieure 2 du boîtier B comporte une face supérieure 2a convexe telle que décrite précédemment et également, comme on peut le voir sur la figure 3, un rebord 2l s'étendant depuis la face supérieure 2a et définissant la surface latérale de la partie supérieure 2. Le rebord 2l s'étend en particulier sur tout le pourtour de la partie supérieure 2 dans cet exemple mais, en variante, le rebord 2l peut ne s'étendre qu'en partie sur le pourtour de la partie supérieure 2 du boîtier B. Le rebord 2l est destiné à être inséré dans la partie inférieure 3 du boîtier B, en prenant appui, notamment du côté intérieur, contre la face latérale 3l de la partie inférieure 3.

La partie supérieure 2 du boîtier B comporte en outre des pattes de fixation 12, comme on peut le voir sur la figure 3, faisant saillies sur le rebord 2l. Les pattes de fixation 12 sont par exemple situées au niveau de coins de la partie supérieure 2. Les pattes de fixation 12 sont notamment destinées à prendre appui sur le crâne C de l'individu, au niveau des portions de crâne situées à la périphérie de la cavité Ca. De la sorte, les pattes de fixation 12 peuvent permettre le maintien du dispositif 1 sur le crâne C en empêchant que celui-ci ne s'enfonce dans le crâne C au niveau de la cavité Ca. Les pattes de fixation 12 peuvent être maintenues sur le crâne C par tout type de fixation, notamment par des vis intracrâniennes. L'épaisseur des pattes de fixation 12 peut être suffisamment faible pour ne pas engendrer de surépaisseur locale à l'interface entre le dispositif 1 et les portions de crâne C adjacentes au dispositif 1 susceptibles d'entraîner des risques de lésions et/ou d'infection de la peau P.

La partie inférieure 3 présente, en plus de sa face inférieure 3b, une face latérale 3l qui définissent entre elles un logement en forme de cuvette pour recevoir les moyens électroniques et optiques 4. La face supérieure 2a de la partie supérieure 2 est destinée à être appliquée contre la peau P du cuir chevelu alors que la face inférieure 3b de la partie inférieure 3 est dirigée vers le cortex du cerveau de l'individu. La partie inférieure 3 peut recevoir le système d'électrodes 5, comme on peut le voir sur la figure 6. La face latérale 3l de la partie inférieure 3 est ajourée. Elle comporte en particulier des ouvertures 11, par exemple de forme oblongue, par exemple au nombre de quatre dans cet exemple. Les ouvertures 11 peuvent permettre le passage de contacts électriques pour coupler électriquement les moyens électroniques et optiques 4 avec le système d'électrodes 5.

La partie supérieure 2 forme un couvercle destiné à venir fermer le logement formé dans la partie inférieure 3 du dispositif 1 par la face latérale 3l et la face inférieure 3b de la partie inférieure 3. En particulier, la partie supérieure 2 peut être soudée sur la partie inférieure 3 du boîtier B.

La partie supérieure 2 et/ou la partie inférieure 3 peuvent présenter une forme sensiblement circulaire, notamment une forme carré. Dans ce cas, les pattes de fixation 12 de la partie supérieure 2 peuvent être situées aux quatre coins de la partie supérieure 2. Des méplats 13 peuvent être formés au niveau de chaque angle de la partie supérieure 2, notamment au niveau de chaque angle du rebord 3l, et les pattes de fixation 12 peuvent faire saillies à partir de ces méplats 13.

La face latérale 3l de la partie inférieure 3 peut également présenter une forme sensiblement circulaire, voire une forme carré. Dans ce cas, les ouvertures 11 peuvent être formées sur chacun des quatre côtés définis par la face latérale 3l.

Le boîtier B, notamment la partie supérieure 2 et/ou la partie inférieure 3, peut être surmoulé par un matériau de surmoulage 10, notamment une résine de silicone biocompatible. Sur la figure 1, la partie inférieure 3 est surmoulée par un tel matériau de surmoulage 10. Le matériau de surmoulage 10 peut permettre de modifier le périmètre externe du boîtier B, et notamment de la partie supérieure 2 et/ou de la partie inférieure 3, par exemple en lui conférant une forme sensiblement cylindrique.

La plus grande dimension transversale de la cavité Ca formée dans le crâne C de l'individu peut être comprise entre 1 et 5 cm. De ce fait, le boîtier B du dispositif implantable 1 peut présenter une plus grande dimension transversale L comprise entre 1 et 5 cm, étant par exemple d'environ 47 mm dans cet exemple. En outre, l'épaisseur E du boîtier B peut par exemple être comprise entre 0,3 et 1 cm, notamment entre 0,5 et 1 cm, par exemple égale à environ 0,7 cm.

Les moyens électroniques et optiques 4 peuvent être logés de façon hermétique à l'intérieur du boîtier B. Les moyens électroniques et optiques 4 peuvent être isolés électriquement du boîtier B.

Les moyens électroniques et optiques 4 peuvent comporter un préamplificateur, un convertisseur analogique numérique et/ou des moyens pour la transmission de données mesurées relatives aux signaux neuronaux.

Les moyens électroniques et optiques 4 peuvent comporter un système d'alimentation et/ou un circuit apte à générer un signal d'alimentation à partir d'un signal détecté par une antenne de téléalimentation.

La figure 6 représente le positionnement du système d'électrodes 5 sous le dispositif implantable 1, au niveau de la face inférieure 3b de la partie inférieure 3 du boîtier B. Ces électrodes 5 sont de préférence réalisées à l'aide d'un matériau conducteur transparent, tel l'ITO (Oxyde d'Indium Etain), déposé sur la face inférieure 3b du boîtier B, en particulier au niveau de sa zone transparente. Ces électrodes 5 sont agencées sur le côté externe de la face inférieure 3b du boîtier B, de façon à être au contact du cortex lorsque le dispositif 1 est implanté.

La figure 7 représente, en vue de face, le système d'électrodes 5. Le système d'illumination 14 n'est pas représenté par souci de clarté. Ces électrodes 5 sont de préférence transparentes.

Le système d'électrodes 5 est ainsi de préférence disposé à l'extérieur du boîtier B. En particulier, la face inférieure 3b du boîtier B et le système d'électrodes 5 peuvent être superposés partiellement, mieux totalement, l'un à l'autre.

Le système d'électrodes 5 comporte de préférence des électrodes du type EcoG utilisant la technologie de l'électrocorticographie décrite précédemment.

Les électrodes du système d'électrodes 5 peuvent être agencées selon une matrice prédéterminée, comme on peut le voir sur la figure 7, par exemple en un réseau de lignes et de colonnes, régulier ou non. En particulier, comme représenté sur la figure 7, le réseau d'électrodes 5 peut être agencé dans une matrice comportant 64 électrodes.

Le système d'électrodes 5 est avantageusement solidaire du boîtier B, étant notamment fixé sur la face inférieure 3l du boîtier B. De la sorte, l'invention peut permettre une maîtrise du positionnement des électrodes sur le cerveau, contrairement aux électrodes selon l'art antérieur réalisées par exemple sur un substrat souple et disposées à distance du boîtier. L'invention peut également permettre d'éviter l'utilisation d'une liaison filaire entre la matrice d'électrodes et le boîtier.

Le système d'électrodes 5 peut être inscrit dans le périmètre externe du boîtier B, notamment dans le périmètre externe de la partie inférieure 3 du boîtier B. De cette façon, il peut y avoir coïncidence entre la zone d'enregistrement des électrodes et la zone accessible du cerveau pour l'enregistrement de signaux neuronaux, formée par la cavité Ca lors d'une craniotomie par exemple.

Selon une variante de réalisation, le système d'électrodes 5 peut comporter des électrodes intracorticales, c'est-à-dire des électrodes aptes à pénétrer dans le cortex, notamment des électrodes reposant sur la technologie LFP décrite précédemment. L'utilisation d'électrodes intracorticales, bien que reposant sur une technologie plus invasive que la technologie de l'électrocorticographie, peut permettre d'obtenir une meilleure résolution spatiale.

En variante encore, le système d'électrodes 5 peut comporter un réseau formé d'électrodes EcoG et d'électrodes intracorticales.

Le positionnement du système d'électrodes 5 peut par exemple être défini en fonction d'examens préalables réalisés sur l'individu.

Le couplage électrique entre le système d'électrodes 5 et les moyens électroniques et optiques 4 logés à l'intérieur du boîtier B peut se faire au niveau de passages hermétiques placés au niveau des ouvertures 11 de la partie inférieure 3 décrites précédemment. Chaque passage hermétique peut comporter un corps métallique, notamment un corps de titane, par exemple une lame, muni d'une ou plusieurs ouvertures. Chaque ouverture peut comporter une broche métallique, notamment réalisée en platine ou platine iridiée, laquelle peut être gainée par du verre ou une céramique, par exemple du rubis de façon à permettre d'assurer l'isolation électrique entre la broche métallique et le corps métallique. Dans l'exemple de réalisation représenté, chaque passage hermétique comporte 23 ouvertures, et donc 23 broches. Au niveau des interfaces entre le corps métallique et le gainage, par exemple entre le titane et le rubis, et entre la broche et le gainage, par exemple entre le platine et le rubis, de l'or peut être déposé de façon à permettre d'assurer l'herméticité. Les passages peuvent en outre être noyés dans un matériau de surmoulage, par exemple une résine époxy ou acrylique isolante.

Selon un autre aspect de l'invention, le dispositif implantable 1 comporte une enveloppe 6 de transmission de signaux. Cette enveloppe 6 a pour fonction la transmission de signaux de et/ou vers les moyens électroniques et optiques 4 par une liaison sans fil.

L'enveloppe 6 peut s'étendre depuis la périphérie du dispositif 1, et notamment depuis la périphérie de la partie inférieure 3 du boîtier B, comme représenté sur la figure 1.

A la différence du boîtier B, l'enveloppe 6 peut être réalisée dans un matériau souple et biocompatible. L'enveloppe 6 peut par exemple être réalisée à partir d'une résine silicone, par exemple la résine silicone commercialisée par la société NUSIL sous la référence MED6210 ou MED6215.

L'enveloppe 6 est destinée à être insérée entre le crâne C et la peau P du cuir chevelu, dans le prolongement de la surface supérieure 2a du boîtier B, comme on peut le voir sur les figures 9 et 10.

L'enveloppe 6 peut présenter une épaisseur e suffisamment mince pour pouvoir être glissée entre le crâne C et la peau P du cuir chevelu. L'épaisseur e de l'enveloppe 6 peut notamment diminuer en éloignement du boîtier B. Autrement dit, l'épaisseur e de l'enveloppe 6 peut diminuer avec la distance par rapport à la périphérie du boîtier B.

L'épaisseur e de l'enveloppe 6 peut par exemple être comprise entre 1 et 3 mm, étant par exemple inférieure à 1,5 mm, mieux 1 mm, mieux encore 0,7 mm. L'épaisseur e de l'enveloppe 6 peut par exemple être constante sur une distance prédéterminée à partir du boîtier B, puis diminuer progressivement jusqu'à l'extrémité de l'enveloppe 6. Dans l'exemple considéré, l'enveloppe 6 présente par exemple une épaisseur e d'environ 1,5 mm sur une distance A maximale de 3 cm par rapport au boîtier B, puis l'épaisseur e diminue progressivement pour atteindre 0.2 mm à l'extrémité de l'enveloppe 6.

L'enveloppe 6 peut présenter une dureté Shore A égale à environ 50. L'enveloppe 6 peut ainsi avoir une souplesse permettant une bonne adaptation lors de l'implantation entre le crâne C et le cuir chevelu.

L'enveloppe 6 peut présenter une surface supérieure 6a sensiblement convexe (ou courbe) de façon à reproduire au moins partiellement la courbure du crâne et celle du cuir chevelu à l'endroit de son implantation, et à assurer une sensible continuité de surface entre la face supérieure 2a du boîtier B et la surface supérieure 6a de l'enveloppe 6. En particulier, la surface supérieure 6a de l'enveloppe 6 peut être reliée à la face supérieure 2a du boîtier B de façon continue de sorte à présenter un arrondi épousant la forme du cuir chevelu et celle du crâne à l'interface entre le boîtier B et l'enveloppe 6. La surface supérieure 6a de l'enveloppe 6 et la face supérieure 2a du boîtier B peuvent être tangentes l'une par rapport à l'autre au niveau de leur interface. L'invention peut ainsi permettre de minimiser les risques de liaisons et/ou d'infection, notamment de la peau, au niveau de l'interface entre l'enveloppe 6 et le boîtier B.

Le dispositif 1 peut en outre comporter au moins une antenne de transmission et/ou au moins une antenne de téléalimentation. Dans cet exemple, l'enveloppe 6 comporte une antenne de transmission 8, pour l'échange d'informations en émission et/ou réception, et une antenne de téléalimentation 7. En particulier, l'enveloppe 6 comporte une antenne de transmission 8 de liaison hertzienne pour communiquer avec l'extérieur. Ces deux antennes sont de préférence coplanaires et disposées de sorte que l'une est inscrite dans l'autre. En particulier, l'antenne de téléalimentation 7 encadre l'antenne de transmission 8, celle-ci étant inscrite dans l'antenne de téléalimentation 7. Le fait d'avoir des antennes coplanaires et inscrites l'une dans l'autre peut permettre de limiter significativement l'épaisseur totale de l'enveloppe 6.

Les antennes sont en outre raccordées aux moyens électroniques et optiques 4 par l'intermédiaire d'une ouverture 11 de la partie inférieure 3 telle que décrite précédemment ou par un passage rendu étanche par une résine isolante biocompatible.

Plus particulièrement, l'enveloppe 6 comporte une antenne de téléalimentation 7 Haute Fréquence (HF) de 13,56 MHz et une antenne de transmission 8 Ultra Haute Fréquence (UHF) de 402 à 405 MHz.

Grâce à l'invention, l'éloignement des antennes contenues dans l'enveloppe 6 par rapport au boîtier B logeant les moyens électroniques et optiques 4 peut permettre de limiter les effets d'écrantage du matériau constituant le boîtier B, c'est-à-dire l'effet d'atténuation du champ électrique en raison de la présence de porteurs de charges électriques mobiles au sein du matériau du boîtier B. En effet, l'enveloppe 6 étant réalisée en un matériau non métallique, par exemple un surmoulage de silicone dans lequel les antennes sont noyées, et le boîtier B étant réalisé en un matériau métallique, notamment en titane, l'éloignement de l'enveloppe 6 par rapport au boîtier B permet de limiter un effet d'écrantage pouvant se produire. En outre, le fait de placer les antennes sous le cuir chevelu peut permettre la transmission d'informations provenant de l'extérieur et/ou en direction de l'extérieur tout en minimisant l'atténuation.

Comme on peut le voir sur la figure 1, la distance maximale I₁ entre le boîtier B et l'extrémité de l'antenne de téléalimentation 7 peut être d'environ 3 cm. De même, la distance maximale b entre le boîtier B et l'extrémité de l'antenne de transmission 8 peut être d'environ 1,5 cm.

La largeur (ou écartement) maximale L₁ de l'antenne de téléalimentation 7 peut être d'environ 4 cm. De même, la largeur maximale L₂ de l'antenne de transmission 8 peut être d'environ 3 cm.

Chaque antenne peut comporter un fil métallique, notamment un fil de platine, d'un diamètre par exemple égal à 500 µm. Chaque antenne peut délimiter une surface donnée. Par exemple, l'antenne de téléalimentation 7 peut s'étendre sur une surface d'environ 10 cm².

Les figures 8a et 8b illustrent respectivement les deux étapes de fabrication de l'enveloppe 6 du dispositif 1 selon l'invention.

Au cours de l'étape illustrée sur la figure 8a, chaque antenne 7 et 8 est recouverte d'un premier surmoulage 9', par exemple à l'aide d'une résine silicone, de façon à rigidifier l'antenne. Le surmoulage 9' peut consister en une gaine d'un diamètre d'environ de 1.5 mm autour du fil constituant l'antenne.

Au cours de l'étape illustrée sur la figure 8b, chaque antenne est recouverte d'un deuxième surmoulage 9, par exemple avec le même matériau que pour le premier surmoulage 9', afin d'obtenir l'enveloppe 6. Le deuxième surmoulage 9 peut être effectué sur l'intégralité des antennes 7 et 8 et de l'espace séparant les antennes 7 et 8.

On va maintenant décrire en référence aux figures 11, 12 et 13 trois variantes de réalisation de dispositifs implantables 1 conformes à l'invention.

Pour ces trois variantes de réalisation, le boîtier de chaque dispositif 1 est monobloc et de même conception que celui décrit précédemment en référence aux figures 1 à 6.

Par ailleurs, dans les exemples de réalisation des figures 11 et 12, les dispositifs 1 comportent chacun une enveloppe 6 comprenant une antenne de téléalimentation 7 et une antenne de transmission 8. Autrement dit, les antennes 7 et 8 sont externes au boîtier B. Dans ce cas, le boîtier B de chaque dispositif 1 est avantageusement réalisé en titane.

Au contraire, dans l'exemple de réalisation de la figure 13, le dispositif 1 est dépourvue d'enveloppe 6, et ainsi de la présence d'antennes externes. En effet, l'antenne de téléalimentation 7 et l'antenne de transmission 8 (non représentées) sont dans cet exemple situées à l'intérieur du boîtier B. Autrement dit, les antennes 7 et 8 sont internes au boîtier B et dans ce cas, le boîtier B de chaque dispositif 1 est avantageusement réalisé en matériau non métallique, par exemple en céramique.

En outre, dans l'exemple de réalisation de la figure 11, le dispositif 1 comporte un système d'illumination 14 du cortex cérébral, interne au boîtier B et comprenant une pluralité de sources lumineuses 14a, notamment des diodes du type LED, disposées selon une matrice. Ces sources lumineuses 14a sont visibles au travers de la zone transparente Z en saphir, ou matériau transparent, formant une fenêtre transparente qui s'étend sur toute la surface de la face inférieure 3b du boîtier B.

Avantageusement, cette zone Z en saphir peut recevoir un traitement antireflet, particulièrement sur le côté interne de la face inférieure 3b, afin de limiter les pertes de lumière par réflexion.

Pour conserver le caractère « biocompatible », le traitement antireflet peut être réalisé par dépôt de silice d'indice 1,33 ou par surmoulage à l'aide d'une silicone de qualité optique, comme celle portant la référence MED 6210 du fabricant NUSIL.

Dans l'exemple de réalisation de la figure 12, le dispositif 1 comporte également un système d'illumination 14 du cortex cérébral, semblable à celui du dispositif 1 de la figure 11. Toutefois, un système d'électrodes 5, comprenant des électrodes 5 disposées en matrice, est également prévu pour permettre la stimulation et/ou l'enregistrement électronique de signaux neuronaux. Ce système d'électrodes 5 est destiné à être en contact avec le cerveau de l'être humain ou animal.

De façon avantageuse, cette matrice d'électrodes 5 est transparente pour ne pas entraver la stimulation optique générée par le système d'illumination 14. En particulier, la matrice d'électrodes 5 peut être réalisée en oxyde d'indium-étain (ou ITO pour « Indium Tin Oxide » en anglais), en nanotubes de carbone, en poly(3,4-éthylènedioxythiophène) (PEDOT), ou encore tout autre matériau conducteur transparent implantable.

Les électrodes 5 et pistes conductrices associées du système d'électrodes 5 peuvent préférentiellement être enrobées dans une ou plusieurs couches minces et transparentes de matériaux de protection pour améliorer leur compatibilité et/ou leur tenue mécanique. De telles couches ont préférentiellement une perméabilité proche de celle rencontrée dans les tissus vivants et la gliose qui se forme au contact des corps étrangers.

Par ailleurs, dans l'exemple de réalisation de la figure 13, le dispositif 1 comporte aussi un système d'illumination 14 du cortex cérébral, semblable à ceux des dispositifs 1 des figures 11 et 12.

De plus, comme indiqué précédemment, dans cet exemple et à la différence des exemples des figures 11 et 12, les antennes de téléalimentation 7 et de transmission 8 sont situées à l'intérieur du boîtier B. Le boîtier B, et notamment sa partie supérieure 2, peut alors être réalisé en céramique de manière à permettre une meilleure transmission. L'épaisseur de la partie supérieure 2 du boîtier B peut alors être comprise entre 1 et 2 mm.

Enfin, dans cet exemple de la figure 13, le dispositif 1 est dépourvu d'un système d'électrodes 5 mais pourrait en variante en être muni, et notamment un système d'électrodes 5 transparentes comme pour l'exemple de la figure 12.

Bien entendu, l'invention n'est pas limitée aux exemples de réalisation qui viennent d'être décrits. Diverses modifications peuvent y être apportées par l'homme du métier.

En particulier, le système d'électrodes 5 peut comporter des électrodes de stimulation, les signaux électriques de stimulation étant alors générés par les moyens électroniques et optiques 4 situés dans le boîtier B. Ces moyens peuvent alors comporter des générateurs de signaux de stimulation.

En outre, de façon préférentielle, le dispositif implantable 1 selon l'invention peut être utilisé pour l'illumination corticale sur une grande surface pour le traitement de la maladie d'Alzheimer. Toutefois, ce dispositif implantable 1 peut également être utilisé pour d'autres types de pathologies.

De plus, pour chacun des exemples de réalisation décrits auparavant, les sources lumineuses 14a peuvent en outre être solidaires individuellement ou collectivement d'un moyen de déplacement, tel un actuateur, et notamment un actuateur piézo-électrique, qui puisse permettre le commandement du déplacement d'une ou de plusieurs sources lumineuses 14a.

L'expression « comportant un » doit être comprise comme étant synonyme de « comportant au moins un », sauf si le contraire est spécifié.

## Revendications

1. Dispositif implantable (1) pour la stimulation optique du cerveau humain ou animal, **caractérisé en ce qu'**il comporte :
- un boîtier (B) destiné à être logé au moins partiellement dans une cavité (Ca) formée sur le crâne (C) d'un être humain ou animal,
- un système d'illumination (14) pour la stimulation optique du cerveau de l'être humain ou animal, situé dans le boîtier (B), et
- des moyens électroniques et optiques (4) situés dans le boîtier (B) pour au moins le contrôle de la stimulation optique du cerveau,
le boîtier (B) comportant une face inférieure (3b), destinée à être positionnée en vis-à-vis du cerveau de l'être humain ou animal, comprenant une zone transparente (Z) pour permettre l'illumination du cerveau.

2. Dispositif selon la revendication 1, **caractérisé en ce que** la zone transparente (Z) de la face inférieure (3b) du boîtier (B) s'étend sur toute la surface de la face inférieure (3b).

3. Dispositif selon la revendication 1 ou 2, **caractérisé en ce que** la zone transparente (Z) de la face inférieure (3b) est réalisée en saphir.

4. Dispositif selon l'une des revendications précédentes, **caractérisé en ce que** le système d'illumination (14) comporte une ou plusieurs sources lumineuses (14a) destinées à illuminer le cortex cérébral de l'être humain ou animal.

5. Dispositif selon la revendication 4, **caractérisé en ce que** la ou les sources lumineuses (14a) sont choisies parmi des diodes électroluminescentes, des diodes électroluminescentes organiques, des diodes laser, des diodes laser à cavité verticale émettant par la surface et/ou une matrice de telles diodes.

6. Dispositif selon la revendication 4 ou 5, **caractérisé en ce que** la ou les sources lumineuses (14a) s'étendent sur toute la surface de la zone transparente (Z) de la face inférieure (3b) du boîtier (B).

7. Dispositif selon l'une des revendications 4 à 6, **caractérisé en ce que** la ou les sources lumineuses (14a) émettent dans l'infrarouge et/ou le proche infrarouge.

8. Dispositif selon l'une quelconque des revendications précédentes, **caractérisé en ce que** la zone transparente (Z) de la face inférieure (3b) du boîtier (B) comporte un diffuseur, notamment un diffuseur intermédiaire ou structuré, pour améliorer l'uniformité d'illumination du cerveau, ou un dispositif optique permettant de focaliser la lumière sur des zones précises.

9. Dispositif selon l'une quelconque des revendications précédentes, **caractérisé en ce que** la zone transparente (Z) de la face inférieure (3b) du boîtier (B) présente une plus grande dimension caractéristique, notamment un diamètre, supérieure ou égale à 1 cm, et de préférence supérieure ou égale à 3 cm, pour permettre une illumination surfacique du cerveau.

10. Dispositif selon l'une quelconque des revendications précédentes, **caractérisé en ce qu'**il est du type interface neuronale pour le traitement de signaux, les moyens électroniques et optiques (4) situés dans le boîtier (B) étant configurés en outre pour le traitement de signaux neuronaux pour la stimulation optique et/ou électrique et l'enregistrement de signaux électriques, et **en ce que** le dispositif (1) comporte un système d'électrodes (5) couplé électriquement aux moyens électroniques (4).

11. Dispositif selon la revendication 10, **caractérisé en ce que** le système d'électrodes (5) comporte une matrice d'électrodes transparente (5) pour permettre la mesure de signaux corticaux, tout en permettant l'illumination du cerveau par le système d'illumination (14).

12. Dispositif selon la revendication 10 ou 11, **caractérisé en ce que** le système d'électrodes (5) comporte des électrodes d'enregistrement de signaux neuronaux et/ou des électrodes de stimulation, et **en ce que** les moyens électroniques et optiques (4) comportent des moyens de traitement de signaux neuronaux mesurés par les électrodes d'enregistrement et/ou des moyens de production de signaux d'électrostimulation par les électrodes de stimulation.

13. Dispositif selon l'une quelconque des revendications précédentes, **caractérisé en ce qu'**il comporte au moins une antenne de transmission (8) et/ou une antenne de téléalimentation (7).

14. Dispositif selon la revendication 13, **caractérisé en ce qu'**il comporte une enveloppe (6) de transmission de signaux, s'étendant depuis la périphérie du boîtier (B), comportant ladite au moins une antenne de transmission (8) et/ou ladite au moins une antenne de téléalimentation (7).

15. Dispositif selon l'une quelconque des revendications précédentes, **caractérisé en ce que** le boîtier (B) comporte une face supérieure (2a), destinée à venir au contact de la peau (P) de la tête de l'être humain ou animal, dont la surface est sensiblement convexe.
